# EUROPÄISCHE PATENTSCHRIFT

(11) **EP 2 883 457 B1**
(45) Veröffentlichungstag und Bekanntmachung des Hinweises auf die Patenterteilung: **15.02.2017**
(21) Anmeldenummer: 13196559.2
(22) Anmeldetag: 10.12.2013
(51) Int. Cl.: A23J 3/34

(54) **Vorrichtung und Verfahren zur kontinuierlichen Herstellung von Proteinhydrolysaten**
Device and process for the continuous production of protein hydrolysates
Dispositif et procédé pour la production continue de hydrolysates de protéineverfahren s

(43) Veröffentlichungstag der Anmeldung: 17.06.2015
(73) Patentinhaber: DMK Deutsches Milchkontor GmbH, 27404 Zeven (DE)
(72) Erfinder: Döring, Sven-Rainer, 27404 Zeven (DE)
(74) Vertreter: Fabry, Bernd

(56) Entgegenhaltungen:
- EP-A1- 2 168 665
- EP-A1- 2 286 900
- EP-A2- 2 615 067
- JP-A- H11 128 693

## Beschreibung

### GEBIET DER ERFINDUNG

Die Erfindung befindet sich auf dem Gebiet der Nahrungsmittel und betrifft ein neues Verfahren zur kontinuierlichen enzymatischen Hydrolyse von Proteinen, sowie eine neue Verwendung eines geeigneten Reaktors.

### STAND DER TECHNIK

Proteinhydrolysate stellen Gemische von Aminosäuren, Dipeptiden und Olgopeptiden mit niedrigem Molekulargewicht bis etwa 1.000 dar, die großtechnisch durch Behandlung von Proteinen mit Alkalien oder vorzugsweise Proteasen hergestellt werden. Je nach Ausgangsstoff handelt es sich bei den Hydrolysaten um Kosmetikrohstoffe oder um Nahrungsmittel; letztere werden auf Basis von Molke oder Milch erhalten. Sie stellen wichtige Zwischenprodukte beispielsweise für die Herstellung von Milchpulvern dar.

Im Nahrungsmittelbereich werden Proteinhydrolysate üblicherweise dergestalt produziert, dass man Proteinkonzentrate, beispielsweise Konzentrate mit einem hohen Gehalt an Molkeproteinen - mit Proteasen wie beispielsweise Pepsin versetzt und bei etwa 30 bis 35 °C hydrolysiert. Bei der Hydrolyse kommt es zu einer Änderung des pH-Wertes, weshalb dem System Regulatoren zugesetzt werden.

So ist beispielsweise aus der EP 0588841 B1 (Danmark Protein) ein Verfahren bekannt, bei dem man Molkenproteinkonzentrate zunächst einer Temperaturbehandlung unterwirft, mit Proteasen behandelt und das Hydrolysat einer Ultrafiltration über eine Membran mit einer Trennschärfe von 10.000 Da unterwirft. Hierbei wandern die Proteinhydrolysate ins Permeat, während die Enzyme im Retentat verbleiben.

Aus der EP 0566877 B1 (Nestle) ist eine Vorrichtung und ein Verfahren bekannt, bei dem man die Hydrolyse in zwei Schritten, nämlich über einen kurzen Zeitraum in einem Kessel und über längere Zeit in einem Strömungsrohr durchführt.

Gegenstand der EP 0671126 B1 (Morinaga Milk) ist die Herstellung von Molkeproteinhydrolysate unter Einsatz eines Enzymcocktails, wobei vor der Hydrolyse Lactose durch Ultrafiltration entfernt und die Produkte nach der Hydrolyse ebenfalls mit Hilfe einer Ultrafiltration von den inaktivierten Enzymen befreit.

Das Problem bei der Herstellung von Proteinhydrolysaten nach dem Stand der Technik besteht indes darin, dass der Fortschritt der Hydrolyse, d.h. der Hydrolysegrad schwer zu verfolgen ist und es daher häufig zu überlangen Reaktionszeiten kommt. Wird der Ansatz sich gewissermaßen selbst überlassen, kann es sein, dass man erst nach Ablassen des Produktes aus dem Reaktor feststellt, dass eine Fehlcharge produziert worden ist. Es liegt auf der Hand, dass es wünschenswert wäre, den Hydrolyseprozess besser überwachen und damit die Reaktionszeiten auch exakt einstellen zu können.

Eine Alternative besteht darin, den Ansatz bei einem Druck von 3 bis 10 bar über eine Ultrafiltrationsmembran zu leiten, die eine Trenngrenze von etwa 500 bis 1.000 Da aufweist. Da Enzyme ein Molgewicht von ca. 25.000 Da und die Proteine ein Molgewicht von etwa 18.000 bis 36.000 Da aufweisen, enthält das auf diese Weise gewonnene Permeat nur noch Hydrolyseprodukt, während das Retentat zurückgeleitet werden kann. Allerdings ist auch dieses Verfahren nicht völlig zufriedenstellend, denn beim Umpumpen werden die Enzyme so hohen Überströmgeschwindigkeiten ausgesetzt, dass die mechanische Belastung zu einer Inaktivierung führt.

Die Aufgabe der Erfindung hat somit darin bestanden, ein verbessertes Verfahren zur enzymatischen Hydrolyse von Proteinen zur Verfügung zu stellen, das insbesondere kontinuierlich betrieben werden kann und die eingangs beschriebenen Nachteile des Stands der Technik, insbesondere die Inaktivierung der Enzyme durch mechanische Beschädigung zuverlässig vermeidet. Ebenfalls Aufgabe der Erfindung war es, eine entsprechende Vorrichtung bereitzustellen, in der das Verfahren durchgeführt werden kann.

### BESCHREIBUNG DER ERFINDUNG

Ein erster Gegenstand der Erfindung betrifft die Verwendung einer Vorrichtung zur Durchführung eines Verfahrens zur Herstellung von Proteinhydrolysaten, wobei die Vorrichtung die folgenden Bauteile umfasst:
(a) einen Reaktionsbehälter zur Aufnahme der zu hydrolysierenden Edukte und der Enzyme **(1)**,
(b) eine Sea Weed-Membran **(2),**
(c) eine Belüftungsvorrichtung **(3),**
(d) eine Vorrichtung zur Absaugung von Flüssigkeiten **(4),**
(e) eine Produktabzugsleitung **(5)** und
(f) eine Eduktzulaufleitung **(6),**
wobei
(i) die Sea Weed-Membran **(2)** im unteren Drittel des Reaktionsbehälters **(1)** angebracht ist,
(ii) die Belüftungsvorrichtung **(3)** sich unterhalb und die Vorrichtung zur Absaugung von Flüssigkeiten **(4)** oberhalb der Sea Weed-Membran **(2)** befinden, und
(iii) die Produktabzugsleitung **(5)** mit der Vorrichtung zur Absaugung von Flüssigkeiten **(4)** verbunden ist.

Die Produktabzugsleitung steht vorzugsweise in direkter Verbindung mit dem Permeatraum der Membran. Die Vorrichtung kann des Weiteren über eine separate Zulaufleitung verfügen, über die beispielsweise eine Pufferlösung zur Einstellung des pH-Wertes eindosiert wird. Weiterhin kann die Vorrichtung über geeignete Mess- und Regeltechnik verfügen.

Ein zweiter Aspekt der Erfindung betrifft ein kontinuierliches Verfahren zur Herstellung von Proteinhydrolysaten, bei dem man in wässriger Lösung Proteine einer enzymatischen Hydrolyse unterwirft, welches sich dadurch auszeichnet, dass man
(i) das Reaktionsgefäß vom Boden aus belüftet und dadurch eine Konvektion erzeugt,
(ii) die durch die Konvektion bewegte Reaktionsmischung durch eine im Reaktionsgefäß befindliche Sea Weed UF-Membran leitet,
(iii) das dabei anfallende Permeat enthaltend die Hydrolyseprodukte unmittelbar nach Durchströmen der Membran absaugt und aus dem Reaktor leitet, während das Retentat, enthaltend nicht umgesetzte Proteine und Enzyme im Reaktionsbehälter verbleibt und
(iv) die Menge an abgezogenem Permeat durch einen kontinuierlichen Zulauf an frischem Ausgangsprodukt ausgleicht.

Überraschenderweise wurde gefunden, dass Sea Weed-Membranen in besonderer Weise geeignet sind, aus einem Gemisch von nichtumgesetzten Proteinen und deren Hydrolyseprodukten sowie den als Katalysatoren verwendeten Enzymen, die Wertprodukte quantitativ abzutrennen. Statt hoher Durchflussgeschwindigkeiten, die zu einer Inaktivierung der Enzyme infolge mechanischer Schädigung führen würde, reicht eine langsame Konvektion, die die Reaktionsmischung umwälzt und dazu führt, dass kontinuierlich Mischung durch die im Reaktionsgefäß befindliche Membran strömt. Dabei wird das die Proteinhydrolysate enthaltende Permeat nach Verlassen der Membran kontinuierlich abgezogen, während nicht umgesetztes Ausgangsmaterial und die Enzyme im Retentat verbleiben und weiter reagieren können. Durch die langsame Konvektion bleiben die Enzyme aktiv und die Menge an abgezogenem Permeat wird durch Zustrom einer gleichen Menge an frischer Lösung der Ausgangsprodukte ersetzt. Auf diese Weise wird sicher vermieden, dass es zu Kavitationseffekten und in der Folge zur Schädigung von Inhaltsstoffen kommt. Auch Beeinträchtigungen, die auf die Scherkraft der Flügelräder von sonst üblicherweise eingesetzten Pumpen zurückzuführen sind, werden vermieden.

Mit Hilfe der erfindungsgemäß verwendeten Vorrichtung und dem erfindungsgemäßen Verfahren werden kontinuierlich Proteinhydrolysate erzeugt, ohne dass ständig frisches Enzym zu dosiert oder die Anlage gewartet werden muss. Die kontinuierliche Fahrweise erlaubt auch durch ständige Probenahme jederzeit den Hydrolysegrad in der abgezogenen Produktmenge feststellen zu können.

### Ausgangsstoffe

Grundsätzlich eignen sich alle gängigen Proteinquellen zur Herstellung der nach dem Verfahren erhältlichen Proteinhydrolysate. Demzufolge kommen pflanzliche wie tierische Rohstoffe in Betracht, wie beispielsweise Weizenproteine, Erbsenproteine, Mandelproteine oder Seidenproteinen; besonders bevorzugt sind indes Molke- oder Milchproteine, da diese für Nahrungsmittelzwecke zugelassen sind. Die Proteine können aus den Ausgangsstoffen nach den dem Fachmann geläufigen Aufarbeitungs- und Extraktionsverfahren gewonnen werden. Vorzugsweise werden wässrige Konzentrate eingesetzt, die einen Proteingehalt von wenigstens 50 Gew.-%, vorzugsweise etwa 60 bis etwa 90 Gew.-% aufweisen. Besonders bevorzugt sind Molken- oder Milchproteinkonzentrate mit einem Proteingehalt von mindestens 60 Gew.-% und vorzugsweise etwa 75 bis etwa 85 Gew.-%.

### Enzyme

Das Hydrolyseverfahren wird üblicherweise unter Einsatz von Proteasen durchgeführt. Zu diesen Enzymen, die die Spaltung der Peptidbindung katalysieren, zählen beispielsweise Akrosin, Aminopeptidase B, Bromelain, Calpain I, Carboxypeptidase A, Cathepsin A, Cathepsin B, Cathepsin D, Cathepsin E, Cathepsin K, Chymotrypsin, Collagenase, Dipeptidylpeptidase 4, Dispase, Elastase, Faktor Ila, Faktor Xa, Ficin, gpr-Endopeptidase, Kallikrein, MBTPS1, Papain, Plasmin, Prepilin Typ IV Peptidase, Prolyl-Oligopeptidase, Proteinase K, Proteasom, Renin, Sekretasen (Alpha-, Beta- und Gamma-Sekretase), Thermolysin, Thrombin, und Urokinase. Bei weitem bevorzugt ist indes der Einsatz von Pepsin und/oder Trypsin. Die Hydrolyse wird vorzugsweise bei einer Temperatur im Bereich des Aktivitätsoptimums der Enzyme durchführt, die in der Regel bei etwa 10 bis etwa 45 °C, vorzugsweise etwa 25 bis etwa 40 °C und insbesondere etwa 30 bis etwa 35 °C liegt.

### Ultrafiltration

Unter Ultrafiltration versteht man eine Filtration durch Membranen mit einer Porengröße < 0,1 µm Es handelt sich um ein rein physikalisches, d.h. mechanisches Membrantrennverfahren, das nach dem Prinzip des mechanischen Größenausschlusses arbeiten: alle Partikel in den Fluiden, die größer als die Membranporen sind, werden von der Membran zurückgehalten. Das wesentliche Merkmal besteht im Sinne der Erfindung darin, dass so genannte Sea Weed-Membranen eingesetzt werden, die bislang nur im Bereich der Abwasseraufbereitung Verwendung gefunden haben. Dabei handelt es sich um Membranen, die die Form von lang gezogenen Fäden aufweisen und in Büscheln angeordnet sind, so dass sie sich wie Seegras in der leichten Strömung des Reaktionssystems bewegen und wegen ihre enormen Oberfläche einen außerordentlich hohen Effizienzgrad aufweisen. Entsprechende Produkte sind von General Electric im Handel erhältlich. Vorzugsweise werden solche Membranen eingesetzt, die eine Trenngrenze von etwa 500 bis etwa 10.000 Da und vorzugsweise von etwa 1.000 bis etwa 5.000 Da aufweisen.

### Verfahrensparameter

Wie erläutert, besteht das Prinzip des erfindungsgemäßen Verfahrens darin, statt mit hohen Überströmgeschwindigkeiten zu arbeiten, bei denen die Enzyme zu Bruch gehen und inaktiviert werden, eine langsame Konvektion im Reaktionsgefäß einzustellen, wobei die Reaktionsmischung in ihrer Strömungsrichtung eine Seaweed-Membran passieren muss. In der Strömung bewegen sich die Fasern der Membran langsam hin und her, was einerseits infolge der hohen Oberfläche zu einer hohen Trennleistung führt, andererseits aber einhergeht mit einer geringen mechanischen Belastung, so dass die Inaktivierung der Enzyme zuverlässig vermieden wird. Die Konvektion wird durch das Belüften der Reaktionsmischung vom Boden aus bewirkt, was gleichzeitig einen selbstreinigenden Effekt zu Folge hat und ein Fouling verhindert. Dazu wird über eine, mehrere vorzugsweise aber eine Vielzahl von Düsen Luft oder ein Inertgas eingeblasen, wobei der Druck in der Regel bei etwa 1,1 bis etwa 1,5 bar und vorzugsweise bei etwa 1,2 bar liegt. Es hat sich zudem als vorteilhaft erwiesen, die Belüftungsdüsen unmittelbar unterhalb der Membran anzubringen und den Zwischenraum nicht größer als 30 cm, vorzugsweise etwa 10 bis etwa 25 cm zu wählen. Der optimale Abstand ist von der Dimension des Reaktionsgefäßes abhängig und kann vom Fachmann ohne Einsatz erfinderischer Tätigkeit eingestellt werden. Unter diesen Umständen wird die Reaktionsmischung nämlich der Membran geradezu zugetrieben, was die Effizienz des Verfahrens erheblich verbessert.

Anstelle der Belüftungsvorrichtung ist es auch möglich, einen Impeller oder eine offen geführte Pumpe einzusetzen, um auf diese Weise eine leichte Strömung bzw. Konvektion über die Membra zu erreichen.

Die Reaktionsmischung, die von der Konvektion getrieben in den Bereich der Seaweed-Membran gelangt wird in ein Permeat und ein Retentat aufgetrennt. Die Proteinhydrolysate, die ein geringes Molekulargewicht haben, treten durch die Ultrafiltrationsmembran hindurch und werden von der Absaugvorrichtung, die einen konstanten Unterdruck von etwa 0,5 bis etwa 0,9 bar und vorzugsweise etwa 0,8 bar erzeugt, angezogen und ausgeschleust, während nicht umgesetzte Proteine und Enzyme im Retentat verbleiben - also schlicht die Ultrafiltrationsmembran nicht passieren - und im Reaktionsraum verbleiben, um dort die Reaktion fortzusetzen.

Auf diese Weise kommt es also dazu, dass beständig ein Strom, der praktisch vollständig aus Proteinhydrolysaten des gewünschten Molekulargewichtes besteht, aus dem Reaktionsbehälter abgezogen wird. Um ein kontinuierliches Verfahren zu gestalten, wird daher eine gleiche frischen Ausgangsproduktes zugeleitet. Der besondere Vorteil dieser Verfahrensführung besteht darin, dass die wesentlichen Schritte - Belüftung des Reaktors zur Herbeiführung einer Konvektion, Ultrafiltration mittels einer Seaweed-Membran und Absaugen des Permeats - alle in der flüssigen Phase, also innerhalb des Reaktionsgefäßes erfolgt. Ein wichtiger Aspekt der Erfindung besteht dabei darin, dass das erfindungsgemäße Verfahren eine Umkehrung der Druckverhältnisse bedingt: die Permeatseite steht unter Unterdruck, so dass das Permeat in den Permeatraum der Membran gezogen wird. Andererseits steht die Retentatseite unter Normaldruck, so dass das Permeat nicht durchgedrückt werden kann.

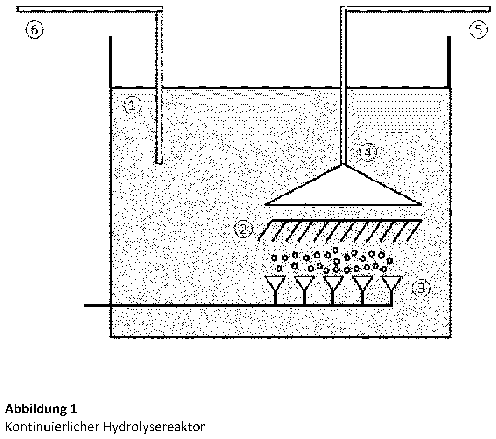

Die erfindungsgemäß verwendete Vorrichtung zur Durchführung des ebenfalls erfindungsgemäßen Verfahrens wird in der **Abbildung 1** näher erläutert. Die dort verwendeten Bezugszeichen finden sich in den nachfolgenden Patentansprüchen wieder.

## Patentansprüche

1. Kontinuierliches Verfahren zur Herstellung von Proteinhydrolysaten, bei dem man in wässriger Lösung Proteine einer enzymatischen Hydrolyse unterwirft, **dadurch gekennzeichnet, dass** man
(i) das Reaktionsgefäß vom Boden aus belüftet und dadurch eine Konvektion erzeugt,
(ii) die durch die Konvektion bewegte Reaktionsmischung durch eine im Reaktionsgefäß befindliche Sea Weed UF-Membran leitet,
(iii) das dabei anfallende Permeat enthaltend die Hydrolyseprodukte unmittelbar nach Durchströmen der Membran absaugt und aus dem Reaktor leitet, während das Retentat, enthaltend nicht umgesetzte Proteine und Enzyme im Reaktionsbehälter verbleibt und
(iv) die Menge an abgezogenem Permeat durch einen kontinuierlichen Zulauf an frischem Ausgangsprodukt ausgleicht.

2. Verfahren nach Anspruch 1, **dadurch gekennzeichnet, dass** man Proteine einsetzt, die ausgewählt sind aus der Gruppe, die gebildet wird von Molkeproteinen, Milchproteinen, Weizenproteinen, Erbsenproteinen, Mandelproteinen und Seidenproteinen.

3. Verfahren nach Anspruch 1, **dadurch gekennzeichnet, dass** man wässrige Proteinkonzentrate einsetzt, die einen Proteingehalt von wenigstens 50 Gew.-% aufweisen.

4. Verfahren nach den Ansprüchen 1 und/oder 2, **dadurch gekennzeichnet, dass** man ein Molken- oder Milchproteinkonzentrat mit einem Proteingehalt von mindestens 60 Gew.-% einsetzt.

5. Verfahren nach mindestens einem der Ansprüche 1 bis 4, **dadurch gekennzeichnet, dass** man als Enzyme Proteasen einsetzt.

6. Verfahren nach Anspruch 5, **dadurch gekennzeichnet, dass** man als Protease Pepsin und/oder Trypsin einsetzt.

7. Verfahren nach mindestens einem der Ansprüche 1 bis 6, **dadurch gekennzeichnet, dass** man das Hydrolyseverfahren bei einer Temperatur im Bereich des Aktivitätsoptimums der Enzyme durchführt.

8. Verfahren nach Anspruch 7, **dadurch gekennzeichnet, dass** man das Hydrolyseverfahren bei etwa 30 bis 35 °C durchführt.

9. Verfahren nach mindestens einem der Ansprüche 1 bis 8, **dadurch gekennzeichnet, dass** man Sea Weed-Membranen einsetzt, die eine Trenngrenze von 500 bis 10.000 Dalton aufweisen.

10. Verfahren nach Anspruch 9, **dadurch gekennzeichnet, dass** man Seaweed-Membranen einsetzt, die eine Trenngrenze von 1.000 bis 5.000 Dalton aufweisen.

11. Verfahren nach mindestens einem der Ansprüche 1 bis 10, **dadurch gekennzeichnet, dass** die Belüftung des Reaktionsgefäßes mit Luft oder einem Inertgas erfolgt, dass mit Hilfe von Düsen eingeblasen wird.

12. Verfahren nach mindestens einem der Ansprüche 1 bis 11, **dadurch gekennzeichnet, dass** die Belüftung mit einem Druck von 1,1 bis 1,5 bar erfolgt.

13. Verfahren nach mindestens einem der Ansprüche 1 bis 12, **dadurch gekennzeichnet, dass** sich die Belüftung unmittelbar unterhalb der Seaweed-Membran befindet und der Zwischenraum nicht mehr als 30 cm beträgt.

14. Verfahren nach mindestens einem der Ansprüche 1 bis 13, **dadurch gekennzeichnet, dass** das Permeat mit einem Druck von 0,5 bis 0,9 bar abgesaugt wird.

15. Verwendung einer Vorrichtung zur Durchführung des Verfahrens nach mindestens einem der Ansprüche 1 bis 14, wobei die Vorrichtung die folgenden Bauteile umfasst:
(a) einen Reaktionsbehälter zur Aufnahme der zu hydrolysierenden Edukte und der Enzyme **(1)**,
(b) eine Sea Weed-Membran **(2),**
(c) eine Belüftungsvorrichtung **(3),**
(d) eine Vorrichtung zur Absaugung von Flüssigkeiten **(4),**
(e) eine Produktabzugsleitung **(5)** und
(f) eine Eduktzulaufleitung **(6),**
wobei
(i) die Seaweed-Membran **(2)** im unteren Drittel des Reaktionsbehälters **(1)** angebracht ist,
(ii) die Belüftungsvorrichtung **(3)** sich unterhalb und die Vorrichtung zur Absaugung von Flüssigkeiten **(4)** oberhalb der Sea Weed-Membran **(2)** befinden, und
(iii) die Produktabzugsleitung **(5)** mit der Vorrichtung zur Absaugung von Flüssigkeiten **(4)** verbunden ist.

## Claims

1. A continuous process for production of protein hydrolysates, in which proteins are submitted to enzymatic hydrolysis in aqueous solution, comprising the following steps:
(i) aerating a reaction vessel containing a reaction mixture of proteins and enzymes from the bottom, thereby producing convection,
(ii) leading the reaction mixture, moving as a result of convection, through a seaweed ultrafiltration membrane present in the reaction vessel,
(iii) withdrawing the resultant permeate containing the hydrolysis products immediately after passing through the membrane and is discharged from the reactor, whereas the retentate, containing unreacted proteins and enzymes, remains in the reaction vessel, and
(iv) compensating the amount of permeate withdrawn by continuous feed of fresh starting proteins.

2. The process according to claim 1, wherein said proteins selected from the group consisting of whey proteins, lactoproteins, wheat proteins, pea proteins, almond proteins and silk proteins.

3. The process according to claim 1, wherein said proteins are aqueous protein concentrates having a protein content of at least 50 weight percent.

4. The process according to claims 1 and/or 2, wherein said protein is a whey or milk protein concentrate having a protein content of at least 60 weight percent.

5. The process according to at least one of claims 1 to 4, wherein said enzymes are proteases.

6. The process according to claim 5, wherein the protease is pepsin and/or trypsin.

7. The process according to at least one of claims 1 to 6, wherein the hydrolysis process is carried out at a temperature in the region of the optimum activity of the enzymes.

8. The process according to claim 7, wherein the hydrolysis process is carried out at about 30 to 35 °C.

9. The process according to at least one of claims 1 to 8, wherein said seaweed ultrafiltration membrane has a size of separation from 500 to 10,000 Dalton.

10. The process according to claim 9, wherein said seaweed ultrafiltration membrane has a size of separation from 1,000 to 5,000 Dalton.

11. The process according to at least one of claims 1 to 10, wherein the aerating of the reaction vessel takes place with air or an inert gas, which is injected by nozzles.

12. The process according to at least one of claims 1 to 11, wherein the aerating takes place with a pressure from 1,1 to 1,5 bar.

13. The process according to at least one of claims 1 to 12, wherein the aerating is located directly beneath the seaweed membrane and the spacing is not more than 30 cm.

14. The process according to at least one of claims 1 to 13, wherein the permeate is withdrawn with a pressure from 0,5 to 0,9 bar.

15. Use of a device for carrying out the process according to at least one of claims 1 to 14, comprising the following components:
(a) a reaction vessel for receiving the educts to be hydrolysed and the enzymes **(1),**
(b) a seaweed membrane **(2),**
(c) an aerating device **(3),**
(d) a device for withdrawing liquids **(4),**
(e) a product discharge line **(5),** and
(f) an educt feed line **(6),**
wherein
(i) the seaweed membrane **(2)** is arranged in the bottom third of the reaction vessel **(1),**
(ii) the aerating device **(3)** is located beneath and the device for withdrawing liquids **(4)** is located above the seaweed membrane **(2),** and
(iii) the product discharge line **(5)** is connected to the device for withdrawing liquids **(4)**.

## Revendications

1. Procédé continu pour la préparation d'hydrolysats de protéines, dans lequel on soumet, en solution aqueuse, des protéines à une hydrolyse enzymatique, **caractérisé en ce que**
(i) on aère le récipient de réaction à partir du fond et on génère ainsi une convection,
(ii) on guide le mélange réactionnel mis en mouvement par la convection à travers une membrane d'UF à base d'algues marines se trouvant dans le récipient de réaction,
(iii) on aspire le perméat produit, contenant les produits d'hydrolyse, immédiatement après l'écoulement à travers la membrane et on le guide hors du réacteur alors que le retentat, contenant des protéines et des enzymes non transformées, reste dans le récipient de réaction et
(iv) on compense la quantité de perméat soutiré par une alimentation continue en produit de départ frais.

2. Procédé selon la revendication 1, **caractérisé en ce qu'**on utilise des protéines qui sont choisies dans le groupe formé par les protéines de lactosérum, les protéines de lait, les protéines de blé, les protéines de pois, les protéines d'amande et les protéines de soie.

3. Procédé selon la revendication 1, **caractérisé en ce qu'**on utilise des concentrats aqueux de protéines qui présentent une teneur en protéines d'au moins 50% en poids.

4. Procédé selon les revendications 1 et/ou 2, **caractérisé en ce qu'**on utilise un concentrat de protéines de lactosérum ou de lait présentant une teneur en protéines d'au moins 60% en poids.

5. Procédé selon au moins l'une quelconque des revendications 1 à 4, **caractérisé en ce qu'**on utilise des protéases comme enzymes.

6. Procédé selon la revendication 5, **caractérisé en ce qu'**on utilise de la pepsine et/ou de la trypsine comme protéase.

7. Procédé selon au moins l'une quelconque des revendications 1 à 6, **caractérisé en ce qu'**on réalise le procédé d'hydrolyse à une température dans la plage de l'optimum d'activité des enzymes.

8. Procédé selon la revendication 7, **caractérisé en ce qu'**on réalise le procédé d'hydrolyse à environ 30 jusqu'à 35°C.

9. Procédé selon au moins l'une quelconque des revendications 1 à 8, **caractérisé en ce qu'**on utilise des membranes à base d'algues marines qui présentent une limite de séparation de 500 à 10.000 daltons.

10. Procédé selon la revendication 9, **caractérisé en ce qu'**on utilise des membranes à base d'algues marines qui présentent une limite de séparation de 1000 à 5000 daltons.

11. Procédé selon au moins l'une quelconque des revendications 1 à 10, **caractérisé en ce que** l'aération du récipient de réaction a lieu avec de l'air ou un gaz inerte qui est insufflé à l'aide de buse.

12. Procédé selon au moins l'une quelconque des revendications 1 à 11, **caractérisé en ce que** l'aération a lieu à une pression de 1,1 à 1,5 bar.

13. Procédé selon au moins l'une quelconque des revendications 1 à 12, **caractérisé en ce que** l'aération se trouve directement sous la membrane à base d'algues marines et l'espace intermédiaire n'est pas supérieur à 30 cm.

14. Procédé selon au moins l'une quelconque des revendications 1 à 13, **caractérisé en ce que** le perméat est aspiré à une pression de 0,5 à 0,9 bar.

15. Utilisation d'un dispositif pour réaliser le procédé selon au moins l'une quelconque des revendications 1 à 14, le dispositif présentant les éléments suivants :
(a) un récipient de réaction **(1)** pour recevoir les produits de départ à hydrolyser et les enzymes,
(b) une membrane **(2)** à base d'algues marines,
(c) un dispositif d'aération **(3),**
(d) un dispositif **(4)** pour l'aspiration de liquides,
(e) une conduite **(5)** de soutirage du produit et
(f) une conduite **(6)** d'alimentation en produit de départ,
(i) la membrane **(2)** à base d'algues marines étant disposée dans le tiers inférieur du récipient de réaction **(1)**,
(ii) le dispositif d'aération **(3)** se trouvant sous et le dispositif **(4)** pour l'aspiration de liquides se trouvant au-dessus de la membrane **(2)** à base d'algues marines et
(iii) la conduite **(5)** de soutirage du produit étant reliée au dispositif **(4)** pour l'aspiration de liquides.
